Europäisches Patentamt

European Patent Office

Office européen des brevets

(11) Veröffentlichungsnummer: **0 494 350 A2**

# EUROPÄISCHE PATENTANMELDUNG

(21) Anmeldenummer: **91118709.4**

(22) Anmeldetag: **02.11.91**

(51) Int. Cl.5: **C07C 31/04**, C07C 29/152

(30) Priorität: **11.01.91 DE 4100632**

(43) Veröffentlichungstag der Anmeldung:
**15.07.92 Patentblatt 92/29**

(84) Benannte Vertragsstaaten:
**CH DE ES FR GB IT LI**

(71) Anmelder: **Uhde GmbH**
**Friedrich-Uhde-Strasse 15**
**W-4600 Dortmund 1(DE)**

(72) Erfinder: **Bähnisch, Hans-Joachim**
**Wembersweg 30**
**W-4600 Dortmund 76(DE)**

(74) Vertreter: **Patentanwälte Meinke und**
**Dabringhaus Dipl.-Ing. J. Meinke Dipl.-Ing. W.**
**Dabringhaus**
**Westenhellweg 67**
**W-4600 Dortmund 1(DE)**

(54) **Verfahren und Anlage zur Methanolsynthese.**

(57) Mit einem Verfahren und einer Anlage zur Methanolsynthese aus Wasserstoff, Kohlenmonoxid und Kohlendioxid unter Druck mit wenigstens einer Stufe, die einen Reaktor enthält, soll die Ausbeute erhöht und die Regelungsmöglichkeit verbessert werden.

Dies wird dadurch erreicht, daß wenigstens zwei Synthesestufen mit je einem Reaktor eingesetzt werden und die letzte Synthesestufe mit einem Kreislaufgas-Verdichter ausgerüstet ist, wobei die vorgeschalteten Synthesestufen zur Bildung von Teilkreisläufen über diesen Kreislaufgas-Verdichter beaufschlagt werden.

Fig. 1

EP 0 494 350 A2

Die Erfindung richtet sich auf ein Verfahren und eine Anlage zur Methanolsynthese aus Wasserstoff, Kohlenmonoxid und Kohlendioxid unter Druck mit wenigstens einer Synthesestufe, die einen Reaktor enthält.

Es sind eine Reihe von Anlagen bzw. Verfahren zur katalytischen Methanolsynthese bekannt, wobei für die Fülle der Lösungen hier als Beispiel die deutschen Offenlegungsschriften 21 17 060, 25 29 591, 32 20 995 und 35 18 362 oder US-29 04 575 genannt seien.

Es ist dabei bekannt, eine Synthesestufe mit einem Reaktor einzusetzen, allenfalls ergänzt durch einen sich selbst regelnden Vorreaktor, ohne daß dieser der bekannten einstufigen Kreislaufführung bei der Methanolsynthese zugeschaltet ist.

Aufgabe der Erfindung ist es, die Ausbeute derartiger Verfahren bzw. Anlagen zu erhöhen und dabei insbesondere für eine gute Regelungsmöglichkeit zu sorgen.

Mit einem Verfahren der eingangs bezeichneten Art wird diese Aufgabe gemäß der Erfindung dadurch gelöst, daß wenigstens zwei Synthesestufen mit je einem Reaktor eingesetzt werden und die letzte Synthesestufe mit einem Kreislaufgas-Verdichter ausgerüstet ist, wobei die vorgeschalteten Synthesestufen zur Bildung von Teilkreisläufen über diesen Kreislaufgas-Verdichter beaufschlagt werden.

Der Vorteil einer solchen Vorgehensweise liegt insbesondere darin, daß durch die Kreislaufführung eine optimale Regelung möglich ist, d.h. die jeweils gewünschten, gleichbleibenden Katalysatorbedingungen aufrechterhalten werden können, was sowohl für das Anfahren einer entsprechenden Anlage gilt wie auch für den Dauerbetrieb.

Durch die Beaufschlagung der Reaktoren mit Kreislaufteilströmen, hervorgerufen durch den letzten Kreislaufgas-Verdichter, ergibt sich auch, daß weniger unerwünschte Nebenprodukte erzeugt werden, wobei die einzelnen Synthesekreisläufe durchaus auch bei unterschiedlichen Drücken betreibbar sind. Der stufenweise Abzug des Produktes aus den einzelnen Synthesestufen bedingt auch kleiner werdende Teilmengen und damit kleiner werdende Anlagenelemente.

In Ausgestaltung ist nach der Erfindung vorgesehen, daß neben dem alle Teilkreisläufe beaufschlagenden letzten Kreislaufgas-Verdichter den vorgeschalteten Stufen zusätzlich eigene Verdichter zugeordnet sind.

Durch diese Verfahrensweise ist es möglich, in den einzelnen Synthesekreisläufen die jeweils gewünschten Drücke einzustellen, die nach Temperatur und Durchsetzmenge durchaus verschieden sein können.

An dieser Stelle sei bemerkt, daß die Mehrfachverdichter auch gerätemäßig von einem einachsigen mehrstufigen Verdichter bereitgestellt werden können, wobei der jeweilige Abgriff des Synthesegases an unterschiedlichen Bereichen des Verdichters erfolgen kann.

Die Erfindung sieht in Ausgestaltung vor, daß neben zwei in Reihe auch parallele Synthesestufen vorgesehen werden, wobei alle Synthesestufen zur Bildung von Teilkreisläufen über den gemeinsamen, der letzten Synthesestufe zugeordneten Kreislaufgas-Verdichter beaufschlagt werden.

Zweckmäßig wird in jeder Synthesestufe ein Produktabzug vorgenommen, wie dies die Erfindung ebenfalls vorsieht, wodurch in der jeweils nachgeschalteten Synthesestufe eine höhere Methanolbildung bewirkt wird und woraus sich von Stufe zu Stufe ein kleiner werdender Synthesegasstrom ergibt, da eine Frischgaszufuhr nur zur ersten Synthesestufe vorgenommen wird.

Zur Lösung der weiter oben angegebenen Aufgabe sieht die Erfindung auch eine Anlage vor, die sich durch wenigstens zwei Methanolreaktoren und wenigstens einen Kreislaufgas-Verdichter in der letzten Synthesestufe und durch diesen Kreislaufgas-Verdichter beaufschlagte Kreislaufgas-Zufuhrleitungen zur Bildung von Teilkreisläufen auszeichnet.

Dabei kann sich erfindungsgemäß eine entsprechende Anlage durch vier Synthesestufen mit vier Methanolreaktoren, diesen zugeordneten Gas/Gaswärmetauschern, Methanolkondensatoren und Methanolabscheidern sowie den Reaktoren zugeordneten Verdichtern, wobei der der letzten Stufen zugeordnete Verdichter als Kreislaufgas-Verdichter aller Stufen ausgebildet ist, auszeichnen, wobei an dieser Stelle bemerkt sei, daß die Vierstufigkeit hier eine der möglichen Gestaltungen darstellt, ohne daß die Erfindung hierauf beschränkt sein soll.

Die Erfindung ist nachstehend anhand der Zeichnung beispielsweise näher erläutert. Diese zeigt in

Fig. 1 ein Anlagenschaltbild nach der Erfindung und in

Fig. 2 ein Anlagenschaubild für eine vierstufige Synthese.

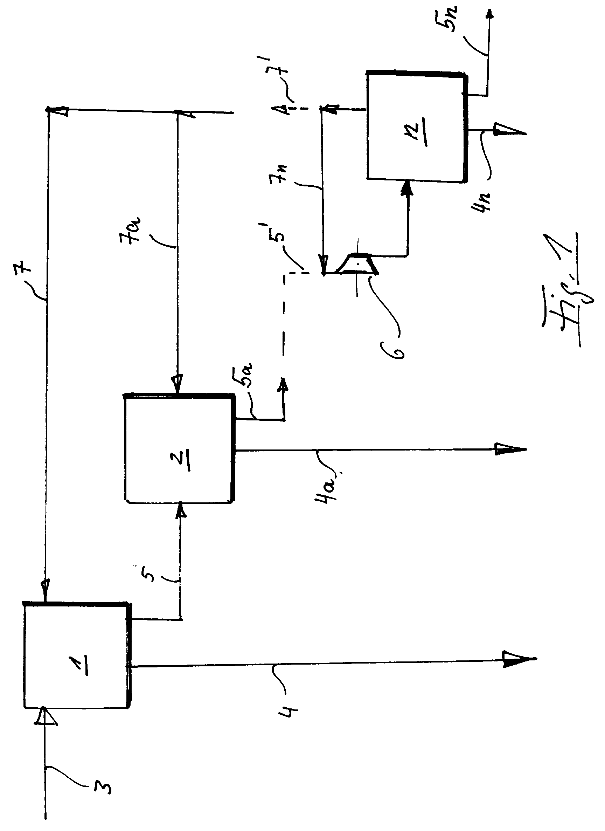

In Fig. 1 ist in vereinfachter Wiedergabe die kaskadenartige Folge einzelner Synthesestufen gemäß der Erfindung dargestellt. Diese Synthesestufen sind mit 1, 2 bzw. "n" bezeichnet. Über die Leitung 3 wird Frischgas der ersten Synthesestufe 1 zugeführt. Die Produktabzugsleitung aus der ersten Synthesestufe ist mit 4 bezeichnet. Das nicht umgesetzte Gas verläßt über die Leitung 5 die erste Synthesestufe und beaufschlagt die zweite Synthesestufe, die Produktabzugsleitung ist hier mit 4a bezeichnet.

Je nach Art der Prozeßführung können noch eine Mehrzahl von Synthesestufen nachgeschaltet sein, bis die letzte Synthesestufe, in Fig. 1 mit "n" bezeichnet, erreicht ist. Das restliche nicht umgesetzte Gas aus der vorletzten Synthesestufe wird einem Kreislaufgas-Verdichter 6 zugeleitet, was über eine gestrichelt dargestellte Leitung 5' dargestellt ist. Über eine Leitung 7 verläßt Kreislaufgas die letzte Synthesestufe "n" und wird im Kreislauf vor den Kreislaufgas-Verdichter 6 geleitet, dies ist mit einer Leitung 7n angedeutet, der Kreislauf zur zweiten Stufe wird über die Leitung 7a geschlossen, während der Kreislauf der ersten Synthesestufe über die Leitung 7 geschlossen wird.

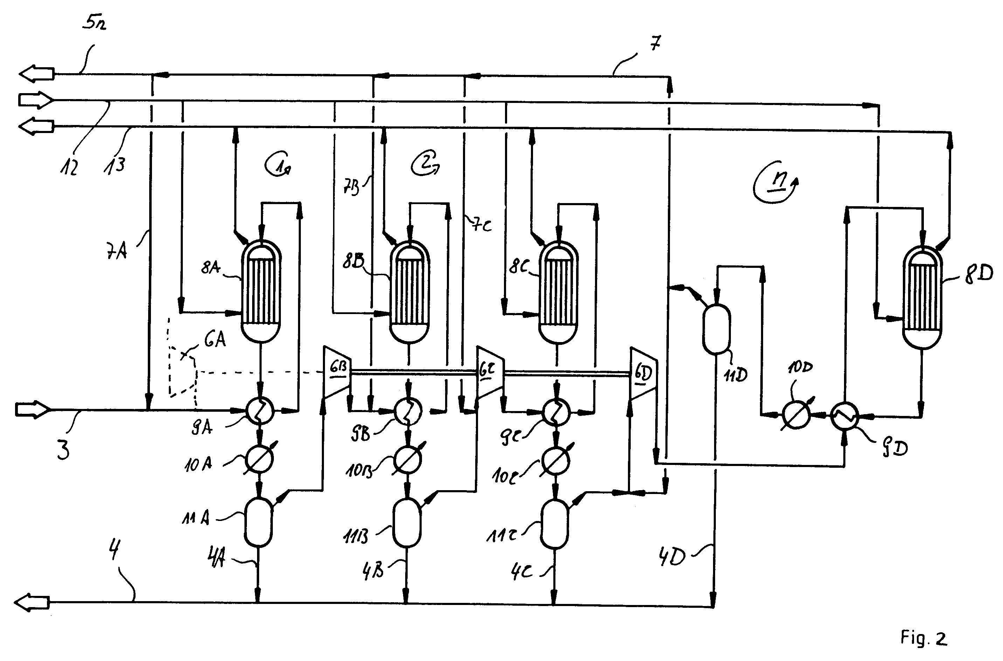

In Fig. 2 ist als Beispiel eine Anlage mit vierstufiger Synthese dargestellt. In jeder Stufe ist ein Methanolreaktor 8, ein Gas/Gaswärmetauscher 9, ein Methanolkondensator 10 und ein Methanolabscheider 11 vorgesehen, die zur einfacheren Identifizierung zusätzlich die Großbuchstaben A bis D tragen, soweit gleich wirkende Bauteile betroffen sind.

Hier dargestellt ist ein dreistufiger Verdichter, dessen auf einer Welle liegende Verdichterstufen mit 6B bis 6D bezeichnet sind, wobei hier angemerkt sei, daß in Fig. 2 links vom Verdichter 6B eine Vorverdichterstufe 6A vorgesehen sein kann, was hier lediglich gestrichelt angedeutet ist.

Soweit wie möglich, sind im übrigen in Fig. 2 noch die Bezugzeichen aus Fig. 1 eingesetzt. Hier zusätzlich angegeben ist noch die Speisewasserzufuhrleitung 12 und die Dampfabführleitung 13, die die Kreisläufe bildenden Leitungen sind hier mit 7 bis 7C bezeichnet.

Nachfolgend wird noch als Beispiel eine Gegenüberstellung einer herkömmlichen Verfahrensweise mit einem einzigen Reaktor zahlenmäßig angegeben sowie eine Kaskade nach der Erfindung mit drei Synthesestufen.

Beispiel Stand der Technik:

Ausgegangen wird von einer Synthese bei 100 bar mit einem Einsatz von 309445 $Nm^3$/h stöchiometrischen Gas mit 5,24 % Inertgehalt. Bei dem herkömmlichen Konzept mit 8 % MeOH im umgesetzten Gas am Reaktoraustritt ergibt sich ein Produktabzug von 99,073 t/h.

Bei einer dreistufigen Kaskade nach der Erfindung ergibt sich nach der ersten Synthesestufe bei gleichem Einsatz von 309445 $Nm^3$/h ein Produktabzug von 32,56 t/h, wobei im umgesetzten Gas am Reaktoraustritt 8,97 % MeOH vorhanden ist, in der zweiten Synthesestufe ein Abzug von 38,38 t/h bei 13,26 % sowie in der letzten Synthesestufe ein Abzug von 37,31 t/h bei 4,5 %, d.h. eine Gesamtmenge von 108,25 t/h. In die erste Synthesestufe wird ein Kreislaufgasstrom von 15472 $Nm^3$/h, in die zweite Synthesestufe wird ein Kreislaufgasstrom von 12208 $Nm^3$/h zurückgeführt. Aus der letzten Synthesestufe werden 47688 $Nm^3$/h Purgegas als verbleibendes, nicht umgesetztes Gas aus der Kaskade abgegeben.

Erkennbar ist die Produktausbeute nach der Erfindung deutlich höher als in dem nach dem Stand der Technik gerechneten Beispiel.

**Patentansprüche**

1. Verfahren zur Methanolsynthese aus Wasserstoff, Kohlenmonoxid und Kohlendioxid unter Druck mit wenigstens einer Stufe, die einen Reaktor enthält,
dadurch gekennzeichnet,
daß wenigstens zwei Synthesestufen mit je einem Reaktor eingesetzt werden und die letzte Synthesestufe mit einem Kreislaufgas-Verdichter ausgerüstet ist, wobei die vorgeschalteten Synthesestufen zur Bildung von Teilkreisläufen über diesen Kreislaufgas-Verdichter beaufschlagt werden.

2. Verfahren nach Anspruch 1,
dadurch gekennzeichnet,
daß neben dem alle Teilkreisläufe beaufschlagenden letzten Kreislaufgas-Verdichter den vorgeschalteten Stufen zusätzlich eigene Verdichter zugeordnet sind.

3. Verfahren nach Anspruch 1 oder 2,
dadurch gekennzeichnet,
daß neben wenigstens zwei in Reihe auch parallele Synthesestufen vorgesehen werden, wobei alle Synthesestufen zur Bildung von Teilkreisläufen über den gemeinsamen, der letzten Synthesestufe zugeordneten Kreislaufgas-Verdichter beaufschlagt werden.

4. Verfahren nach einem der vorangehenden Ansprüche,
dadurch gekennzeichnet,
daß in jeder Synthesestufe über einen Methanolabscheider ein Produktabzug vorgenommen wird.

5. Anlage zur Methanolsynthese aus Wasserstoff, Kohlenmonoxid und Kohlendioxid unter Druck, zur Durchführung des Verfahrens nach einem der vorangehenden Ansprüche,
gekennzeichnet durch
wenigstens zwei Methanolreaktoren (8) und wenigstens einen Kreislaufgas-Verdichter (6) in der letzten Synthesestufe und durch diesen Kreislaufgas-Verdichter (6) beaufschlagbare Kreislaufgas-Zufuhrleitungen (7) zur Bildung von Teilkreisläufen (1,2,n).

6. Anlage nach Anspruch 5,

gekennzeichnet durch
vier Synthesestufen mit vier Methanolreaktoren (8), diesen zugeordneten Gas/Gaswärmetauschern (9), Methanolkondensatoren (10) und Methanolabscheidern (11) sowie den Reaktoren zugeordneten Verdichtern (6), wobei der der letzten Stufe zugeordnete Verdichter (6D) als Kreislaufgas-Verdichter aller Stufen ausgebildet ist.

Fig. 1

Fig. 2

EP 0 494 350 A2